# EUROPEAN PATENT SPECIFICATION

(11) **EP 0 587 925 B1**
(45) Date of publication and mention of the grant of the patent: **04.12.1996**
(21) Application number: 92115712.9
(22) Date of filing: 14.09.1992
(51) Int. Cl.: A61F 11/12

(54) **Soiling resistant earplug**
Verschmutzungsbeständiger Ohrstöpselbügel
Bouchon auriculaire ayant une monture évitant la souillure

(43) Date of publication of application: 23.03.1994
(73) Proprietor: LEIGHT & ASSOCIATES, Santa Monica, CA 90404 (US)
(72) Inventor: Leight, Howard S., Santa Monica, CA 90404 (US)
(74) Representative: Schaumburg, Thoenes & Thurn

(56) References cited:
- FR-A- 1 567 666
- US-A- 2 395 356
- US-A- 3 735 836
- US-A- 3 895 627
- US-A- 3 943 925
- PATENT ABSTRACTS OF JAPAN vol. 9, no. 321 (E-367)(2044) 17 December 1985 & JP-A-60 153 226 (TAKASHI HIRAMATSU)

## Description

One type of earplug apparatus includes a resilient band that fits halfway about the head of the wearer and which has opposite ends holding earplug devices that press against the wearer's ears. Such banded earplugs are used in large numbers in manufacturing and other industries to protect against loud noises. When a worker removes a banded earplug, he often lays it on a nearby horizontal surface such as that of a table, shelf, or desk. The horizontal surface may be soiled from dust, grease, or other contaminants in the environment, and may soil the earplug devices that touch them. This can lead to dirtying the worker's ears or to the worker often requiring a new clean banded earplug. An example of prior art devices is the stethoscope described in FR-A-1 567 666.

The problem to be solved by the invention is to provide a band earplug apparatus which ensures reduced soiling of the earplug devices.

The invention solves this problem by the features of the independent claim. An advantageous further development is specified in the subclaim.

In accordance with one embodiment of the present invention, a banded earplug is provided which avoids soiling of its earplug devices when the banded earplug is laid on a largely horizontal surface. The banded earplug includes a band having a middle portion formed to rest stably on a horizontal surface, and having end portions that extend away from the horizontal surface. The earplug devices are mounted at locations on the end portions furthest from the middle portion, so they are held away from the horizontal surface to avoid soiling. The middle portion of the band can form intermediate portions that each includes a pair of spaced bearing locations that rest on the horizontal surface and a raised portion between the bearing locations. The raised portion helps assure stable resting of the earplug even on an uneven largely horizontal surface.

The invention will be best understood from the following description when read in conjunction with the accompanying drawings in which:
Fig. 1 is an isometric view of a banded earplug apparatus of the present invention, showing it worn by a person,
Fig. 2 is a plan view of the band earplug apparatus of Fig. 1,
Fig. 3 is a view taken on the line 3 - 3 of Fig. 2, and
Fig. 4 is a side elevation view of the apparatus of Fig. 1, worn on the person.

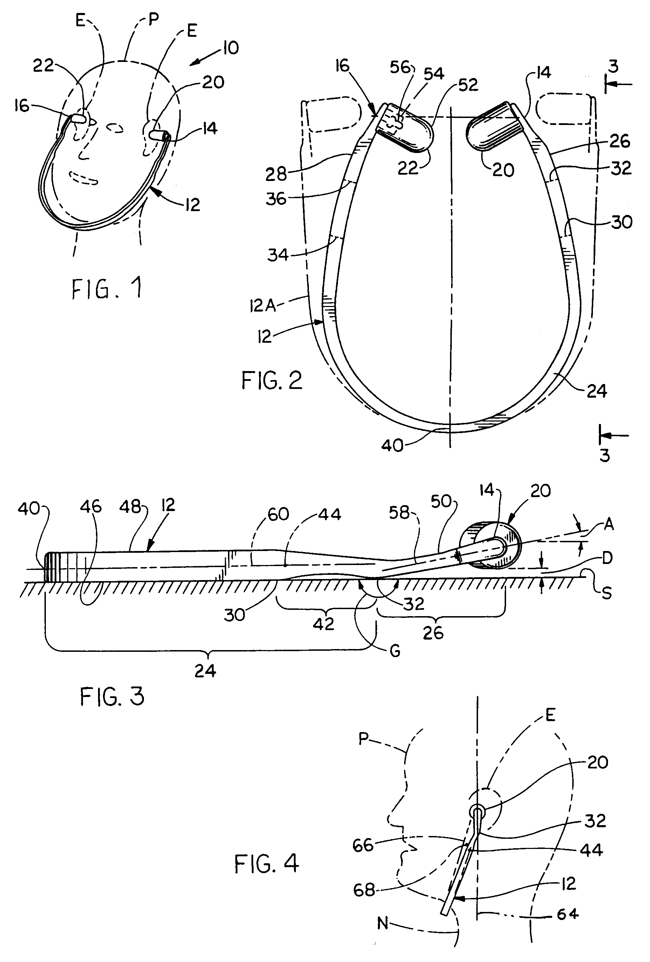

Fig. 1 illustrates a banded earplug 10 which includes a resilient band 12 designed to extend about halfway around the head of a person P, and which has opposite ends 14, 16 that lie opposite the ears E of the person. A pair of earplug devices 20, 22 are each attached to a different one of the band ends. The resilient band 12 urges the earplug devices 20, 22 towards the person's ears, so each device presses against the entrance to the ear canal of the person, to form a seal which minimizes the passage of noise to the person's ear.

Banded earplugs of this general type are well known, and are commonly used in workplaces such as factories, where they are reused many times before disposal. Workers commonly remove the earplugs for extended periods such as during coffee and lunch breaks, when the banded earplugs must be stored. Such storage is commonly achieved by merely laying the banded earplug on an easily available surface such as an unused region of a table. Since surfaces in manufacturing plants often become dirty from oil, bits of metal that have been ground or machined from work pieces, and other soiling material, the earplug devices often become soiled. If a workman places a soiled earplug against his ears, the soiling material may harm the worker, or at least feel uncomfortable. The alternative is for the worker to request another banded earplug, which results in additional expense.

In accordance with the present invention, the banded earplug 10 is constructed so that when laid on a largely flat and horizontal surface S such as shown in Fig. 3, the earplug devices such as 20 are held away from the surface, to thereby avoid soiling of the earplug devices when the horizontal surface S is dirty. The band 12 includes a middle portion 24 and opposite end portions 26, 28. The middle portion 24 is designed to rest stably on the surface S, with the end portions extending at an incline angle A. The incline brings the band ends 14, 16 sufficiently away from the surface S to hold the earplug devices spaced a distance D away from the surface.

The middle portion of the band includes a pair of bearing locations 30, 32 and 34, 36 which are designed to rest on a horizontal surface. Each pair of bearing locations includes an inner bearing location such as 30 closest to the center 40 of the middle portion 24 of the band, and a second or outer bearing location such as 32 furthest from the center 40. The band middle portion also preferably includes a recessed or raised portion 42 extending between each pair of bearing locations 30, 32. The raised portion 42 helps assure that the band will lie stably on a surface with the band ends 14, 16 far enough from the surface to avoid contact of the earplug devices with the surface, even if the surface has moderate irregularities. The center of gravity of the banded earplug is at the location 44 which is preferably located closer to the inner bearing location 30 than to the outer one 32, and which is generally located at about the inner location 30.

The band has lower and upper faces 46, 48 and it is fairly obvious to the worker which face 46 is to be laid on the surface to avoid soiling of the earplug devices. Instructions provided with each banded earplug point this out to the worker. The worker can be encouraged to grasp the banded earplug at the end portions such as 26 near the inclined regions 50, to facilitate lay-down of the banded earplug on a surface.

In one banded earplug design by applicant, each earplug device such as 22 shown in Fig. 2, includes an earplug body 52 of slow recovery foam material, of a diameter of about 18mm so that it presses and seals against the entrance to the ear canal without substantially entering the ear canal. The body 52 is held on a foam armature 54 anchored by a post 56 molded integrally with the end 16 of the band. The band has to be spread apart to the configuration 12A to fit around the person's head, and then applies a force of about 170 g (6 ozs.) to the ears of the person. As shown in Fig. 3, the band end portions such as 26, extend along an imaginary line 58 angled at an angle A of about 11° from the length of the middle portion of the earplug, as indicated by an imaginary line at 60. Applicant prefers to maintain the angle A less than about 45°, because a large angle results in an earplug of somewhat greater bulk and therefore somewhat greater expense, and because a small angle is sufficient to keep the devices away from the horizontal surface and has a largely straight aesthetic appearance. The angle A is generally at least 10°.

The construction of the band, with the end portions such as 26 angled from the middle portion 24, also aids in comfortable wearing of the band earplug. As shown in Fig. 4, the banded earplug is preferably worn with the convex side (i.e., forming an angle G of over 180°) at location 32 facing rearwardly. This results in the center of gravity 44 of the band earplug (as seen in the side views of Figs. 3 and 4) lying closer to a vertical line 64 passing through the earplugs such as 20. This may be compared to a straight band indicated at 66 where the center of gravity 68 lies further from the vertical line. As a result, in the present band 12 there is less gravity-caused movement tending to pivot the banded earplug more toward the vertical (wherein the center of gravity 44 lies on line 64), and the banded earplug is less likely to slip and press against the neck N of the wearer and discomfort him. The distance between the centers of gravity 44 and 68 increases as the tilt angle A increases.

Thus, the invention provides a band earplug apparatus or banded earplug which avoids soiling of the earplug devices that press against the wearer's ears, when the banded earplug is laid on a largely horizontal surface for storage. The banded earplug includes a band with a middle portion formed to rest stably on a flat horizontal surface and end portions that extend at an angle to the middle portion to hold the earplug devices away from the support surface. The middle portion can be formed with pairs of bearing locations that normally rest on a horizontal surface and can be formed with a recessed or raised portion between the bearing locations, to help avoid unwanted tilting of the earplug if there is a bump on the support surface under the raised portion of the band. The end portions of the band preferably extend at a small to moderate incline from a horizontal surface on which the middle portion of the band rests.

## Claims

1. A band earplug apparatus comprising:
a band (12) which can extend about halfway around a person's head, and which has a middle portion (24) and opposite ends that can lie opposite the ears of the person, the opposite ends extending away from the largely horizontal rest surface of the middle portion (24);
a pair of earplug devices (20, 22), each attached to a different one of said band ends, and said band being constructed to urge said band ends and the earplug devices thereon toward the person's ears; characterized by
the middle portion (24) of said band (12) extending generally along an imaginary line (60) as viewed in a side view, and the end portions (26, 28) each extending at an angle (A) of at least 10° away from said imaginary line (60), said middle and end portions (24; 26, 28) having a convex location (32) where they meet, whereby to help keep the band (12) away from the wearer's neck when the apparatus is worn with said convex location (32) facing rearwardly of the wearer.

2. The apparatus described in claim 1 wherein:
said band middle portion (24) includes two pairs of bearing locations (30, 32; 34, 36), each of said pairs of bearing locations includes first locations (30, 34) and second locations (32, 36) respectively closest to and furthest from said band center, said band (12) having a raised region (42) between each of said first and second bearing locations (30, 32; 34, 36), said raised region (42) lying above a horizontal support surface (S) when said bearing locations (30, 32; 34, 36) lie thereon.

## Patentansprüche

1. Ohrstöpselbügel-Vorrichtung, umfassend:
- einen Bügel (12), der sich etwa halb um den Kopf einer Person erstrecken kann und der einen mittleren Bereich (24) und entgegengesetzte Enden hat, die dem Ohr der Person gegenüberliegen können, wobei sich die entgegengesetzten Enden von der weitgehend horizontalen Auflagefläche des mittleren Bereichs (24) weg erstrecken:
- ein Paar von Ohrstöpselelementen (20, 22), deren jedes an einem anderen der Bügelenden angebracht ist, wobei der Bügel derart ausgebildet ist, daß er die Bügelenden und die dort befestigten Ohrstöpselelemente in Richtung auf die Ohren der Person drückt;
dadurch gekennzeichnet, daß sich der mittlere Bereich (24) des Bügels (12) aus seitlicher Sicht allgemein entlang einer imaginären Linie (60) erstreckt und daß sich die Endbereiche (26, 28) jeweils in einem Winkel (A) von wenigstens 10° von der imaginären Linie (60) weg erstrecken, wobei der mittlere Bereich und die Endbereiche (24; 26, 28) eine konvexe Stelle (32) aufweisen, an der sie sich treffen, wodurch dazu beigetragen wird, den Bügel (12) von dem Hals des Trägers fernzuhalten, wenn die Vorrichtung so getragen wird, daß die konvexe Stelle (32) vom Träger nach hinten weist.

2. Vorrichtung nach Anspruch 1, bei welcher der mittlere Bereich des Bügels (24) zwei Lagerstellenpaare (30, 32; 34, 36) hat, deren jedes erste Stellen (30, 34) und zweite Stellen (32, 36) aufweist, die am nächsten zur bzw. am entferntesten von der Bügelmitte gelegen sind, wobei der Bügel (12) zwischen jeweils jeder ersten und zweiten Lagerstelle (30, 32; 34, 36) einen erhöhten Bereich (42) hat, der über einer horizontalen Lagerfläche (S) liegt, wenn diese Lagerstellen (30, 32; 34, 36) auf dieser Lagerfläche liegen.

## Revendications

1. Bouchon auriculaire avec monture comprenant:
une monture (12) qui est apte à s'étendre environ autour de la moitié de la tête d'une personne, et qui possède une portion intermédiaire (24) et des extrémités en vis-à-vis qui sont aptes à se placer en face des oreilles de la personne, les extrémités en vis-à-vis s'écartant de la surface d'appui essentiellement horizontale de la portion intermédiaire (24);
une paire d'éléments formant bouchons auriculaires (20, 22), chacun d'eux étant fixé à une extrémité différente desdites extrémités de monture, et ladite monture étant conçue pour presser lesdites extrémités de monture et les éléments formant bouchons auriculaires placés sur celles-ci en direction des oreilles de la personne; caractérisé par le fait que :
la portion intermédiaire (24) de ladite monture (12) s'étend d'une manière générale le long d'une ligne imaginaire (60) en vue latérale, et les portions d'extrémité (26, 28) s'écartent toutes les deux selon un angle (A) d'au moins 10° de ladite ligne imaginaire (60), lesdites portions intermédiaire et d'extrémité (24; 26, 28) se rejoignant à un emplacement convexe (32), pour aider par voie de conséquence à maintenir la monture (12) écartée du cou du porteur lorsque l'appareil est porté avec ledit emplacement convexe (32) tourné vers l'arrière du porteur.

2. Appareil selon la revendication 1, dans lequel:
la portion intermédiaire de monture (24) comprenant deux paires d'emplacements d'appui (30, 32; 34, 36), chacune desdites paires d'emplacements d'appui comprenant un premier emplacement (30; 34) et un second emplacement (32; 36) respectivement plus proche et plus éloigné du centre de ladite monture, et ladite monture (12) possédant une région rehaussée (42) entre chacun desdits premier et second emplacements d'appui (30, 32; 34, 36), ladite région rehaussée (42) étant située au-dessus d'une surface de support horizontale (5) lorsque lesdits emplacements d'appui (30, 32; 34, 36) reposent sur elle.
